(19)

Europäisches
Patentamt
European
Patent Office
Office européen
des brevets

(11) **EP 2 223 712 A1**

(12) **EUROPEAN PATENT APPLICATION**
published in accordance with Art. 153(4) EPC

(43) Date of publication:
**01.09.2010 Bulletin 2010/35**

(51) Int Cl.:
*A61M 1/02* (2006.01)    *B01D 15/00* (2006.01)

(21) Application number: **08868804.9**

(22) Date of filing: **26.12.2008**

(86) International application number:
**PCT/JP2008/073685**

(87) International publication number:
**WO 2009/084613 (09.07.2009 Gazette 2009/28)**

(84) Designated Contracting States:
**AT BE BG CH CY CZ DE DK EE ES FI FR GB GR
HR HU IE IS IT LI LT LU LV MC MT NL NO PL PT
RO SE SI SK TR**
Designated Extension States:
**AL BA MK RS**

(30) Priority: **27.12.2007 JP 2007335868**

(71) Applicant: **Toray Industries, Inc.
Tokyo, 103-8666 (JP)**

(72) Inventors:
• **IWANAGA, Ema
  Otsu-shi
  Shiga 520-8558 (JP)**
• **SHIMAGAKI, Masaaki
  Otsu-shi
  Shiga 520-8558 (JP)**

(74) Representative: **Kador & Partner
Corneliusstrasse 15
80469 München (DE)**

(54) **FIBER CONSTRUCT FOR TREATING BIOLOGICAL COMPONENTS**

(57)    This object aims to provide a fiber construct which is made up of ultrafine fibers and yet can stably sustain voids among fibers even in the case of being packed into a small-sized column. Namely, a fiber construct for treating biological components which is made up of fibers having an average diameter of less than 50 [mu]m and in which a part of the above-described fibers are curled.

EP 2 223 712 A1

**Description**

TECHNICAL FIELD

The present invention relates to a fiber construct for treating biological components.

BACKGROUND ART

Blood cell apheresis is carried out by passing the blood through a column in which a fiber construct is packed thereby removing excessive leukocytes from the blood, and is a known therapy for autoimmune diseases such as chronic articular rheumatism, which is likely due to cell disruption or damage caused by abnormally activated or proliferated leukocytes (Patent Documents 1 and 2).

Also known are columns for adsorbing cytokines (Patent Documents 3 and 4) and columns for adsorbing leukocytes and toxins (Patent Document 5). From the viewpoints of handling easiness and reduction of loads on pediatric patients, miniaturization of these columns has been desired.

Patent Document 1: Japanese Unexamined Patent Application Publication No. 60-193468
Patent Document 2: Japanese Unexamined Patent Application Publication No. 5-168706
Patent Document 3: Japanese Unexamined Patent Application Publication No. 10-225515
Patent Document 4: Japanese Unexamined Patent Application Publication No. 12-237585
Patent Document 5: Japanese Unexamined Patent Application Publication No. 2002-113097

DISCLOSURE OF INVENTION

PROBLEMS TO BE SOLVED BY THE INVENTION

The use of ultrafine fibers having a large surface area and a diameter of about 0.5 to 20 $\mu$m is effective for the miniaturization of the columns. However, fibers with a too small diameter have poor firmness, and thus cannot stably keep gaps between the fibers.

In consideration of the above-described problems, the present invention is intended to provide a fiber construct composed of ultrafine fibers, the fiber construct keeping its form so as to be suitable for treating biological components even when packed into a small column having a limited volume.

MEANS FOR SOLVING THE PROBLEM

As a result of intensive research for solving the above-described problems, the inventors found a fiber construct suitable for treating biological components, and thus have accomplished the present invention.

More specifically, the present invention includes the following aspects 1 to 7.

1. A fiber construct for treating biological components, including fibers having an average diameter of less than 50 $\mu$m, some of the fibers being crimped.
2. The fiber construct of the aspect 1, which is used as a medical material.
3. The fiber construct of the aspect 1 or 2, wherein the average diameter of the fibers is 0.5 $\mu$m or more and less than 10 $\mu$m, and the amplitude of the crimps is 5 to 200 $\mu$m.
4. The fiber construct of any one of the aspects 1 to 3, wherein the coefficient of variation for the amplitude is 0.1 or more.
5. The fiber construct of any one of the aspects 1 to 4, which has any form selected from the group consisting of nonwoven fabric, knitted fabric, woven fabric, and cotton.
6. The fiber construct of any one of the aspects 1 to 5, wherein the biological component is blood, or the fiber construct is used for treating blood.
7. A column for treating biological components packed in which fiber construct of any one of the aspects 1 to 6 is packed.

ADVANTAGEOUS EFFECT OF THE INVENTION

The fiber construct according to the present invention is composed of ultrafine fibers, and has a large surface area in spite of its small volume. Since some of the fibers are crimped so as to improve the firmness of the fiber construct, the fiber construct is suitable as a packing for a small column for treating biological components.

BRIEF DESCRIPTION OF THE DRAWINGS

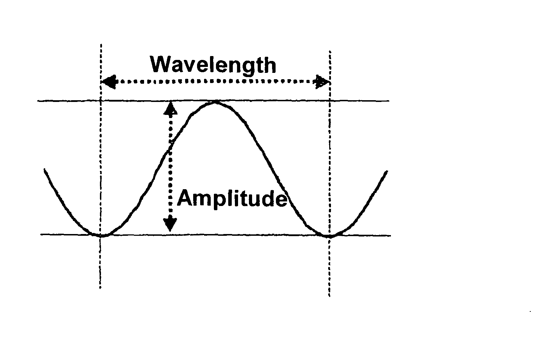

Fig. 1 shows the amplitude and wavelength of a crimp of a fiber composing the fiber construct.

BEST MODE FOR CARRYING OUT THE INVENTION

Preferred embodiments of the present invention are described below.
The fiber construct of the present invention for treating biological components is composed of fibers having an average diameter of less than 50 $\mu$m, some of the fibers being crimped.
The term "biological components" refers to fluids and other components occurring in human and animal bodies, such as blood, lymph, and tissue fluids.
The "average diameter of fibers" is determined as follows. Ten small pieces are randomly taken from the fiber construct, and photographed with a scanning electron microscope at a magnification of 1000 to 3000. The diameter of the fibers is measured at 10 points in each photograph (100 points in total), and the measurements are averaged.
The term "crimped" means that the fibers are crimped at a specific amplitude and wavelength.
The "amplitude" is determined as follows. Ten small sample pieces are randomly taken from a fiber construct, and photographed with a scanning electron microscope at a magnification of 50 to 200. The distance from the top of a peak to the bottom of a valley of a crimped fiber is measured at two points in each photograph (20 points in total), and the measurements are averaged. The "wavelength" is determined by measuring the length from the top of one peak to the next (Fig. 1) at two points in each photograph (20 points in total), and averaging the measurements. The "coefficient of variation" for amplitude is determined by dividing the standard deviation of the measurements for calculating the amplitude by the average.
The fiber construct is preferably in the form of nonwoven fabric, knitted fabric, woven fabric, or cotton, and more preferably in the form of nonwoven fabric or cotton thereby increasing the area to be brought into contact with biological components. When the fiber construct is packed into a radial flow column such as TORAYMYXIN (registered trademark), the fiber construct is preferably in the form of nonwoven fabric from the viewpoint of firmness.
The thickness of the fiber construct in the form of nonwoven fabric is preferably 0.01 to 10 cm from the viewpoint of handling easiness.
The average diameter of the fibers composing the fiber construct must be less than 50 $\mu$m for facilitating the removal of cells and other components from the blood. The average diameter is preferably from 0.5 to 30 $\mu$m, more preferably from 0.5 to 20 $\mu$m, and even more preferably from 0.5 to 10 $\mu$m. The average diameter of the fibers is preferably from 5 to 10 $\mu$m for selectively removing granulocytes from the blood, and is preferably from 0.5 to 4 $\mu$m for efficiently removing granulocytes and lymphocytes from the blood.
When the fiber construct includes crimped fibers, the firmness of the fiber construct is enhanced even if its average diameter is small. Therefore, the proportion of the crimped fibers contained in the fiber construct is preferably 10 wt% or more, more preferably 30 wt% or more, and even more preferably 50 wt% or more.
The firmness deteriorates if the amplitude of the crimps is more than 200 $\mu$m, and clogging tends to occur if the amplitude is less than 5 $\mu$m. Therefore, the amplitude of the crimps is preferably from 5 to 200 $\mu$m, more preferably from 10 to 100 $\mu$m, and even more preferably about 50 $\mu$m on average.
The coefficient of variation for the amplitude is preferably 0.1 or more, and is more preferably 0.4 or more for adsorbing a substance having varied particle sizes, such as leukocytes (6 to 15 $\mu$m).
The firmness deteriorates if the wavelength of the crimps is more than 300 $\mu$m, and clogging tends to occur if the wavelength is less than 10 $\mu$m. Therefore, the wavelength of the crimps is preferably from 10 to 300 $\mu$m, and more preferably 40 to 200 $\mu$m.
The crimps may be waved, coiled, spiraled, serrated, or angular. These forms may be randomly mixed.
The fibers composing the fiber construct are preferably made of a polymer containing an amine residue fixed as a functional group. The polymer more preferably includes a quaternary ammonium group and/or a primary to tertiary amino group or a linear amino group fixed (hereinafter referred to as a quaternary ammonium group or the like).
Examples of the reactive functional group for fixing the quaternary ammonium group or the like include active halogen groups such as halomethyl groups, haloacetyl groups, haloacetamidemethyl groups, and alkyl halide groups, epoxide groups, carboxyl groups, isocyanate groups, thioisocyanate groups, and acid anhydride groups. Among them, active halogen groups are preferred, and haloacetyl groups are more preferred from the viewpoint of the fixing reaction conditions and the stability of the covalent bonds to be formed.
The primary to tertiary amino group preferably has 18 or less carbon atoms per nitrogen atom, and more preferably 3 to 18 carbon atoms per nitrogen atom. In order to adsorb cytokines, the tertiary amino group preferably includes an alkyl group having 4 to 14 carbon atoms.

Examples of the linear amino group include tetraethylenepentamine, and examples of the tertiary amino group include trimethylamine, triethylamine, N,N-dimethylhexylamine, N,N-dimethyloctylamine, N,N-dimethyllaurylamine, and N-methyl-N-ethyl-hexylamine.

If the fixation density of the quaternary ammonium group or the like is too low, the group tends not to exhibit its function. On the other hand, if the fixation density is too high, the physical strength of the fiber construct tends to deteriorate. Therefore, the fixation density is preferably from 0.01 to 2.0 moles, and more preferably from 0.1 to 1.0 mole for one repeating unit of a polymer.

Examples of the method for fixing a quaternary ammonium group or the like in a polymer include the reaction using potassium iodide as a catalyst, and the method including immersing a fiber construct made of nonwoven fabric in a solution, which has been prepared by dissolving a polymer containing an amine residue such as a quaternary ammonium group or the like in a solvent (e.g., methylene chloride, tetrahydrofuran, or N,N-dimethylformamide), followed by evaporation removal of the solvent.

It is also preferred that a hydrophobic group be fixed in place of or in addition to the amine residue in the fibers composing the fiber construct. Examples of the hydrophobic group include alkyl groups such as an ethyl group, an octyl group, a hexyl group, or a lauryl group, and groups containing an aromatic ring.

Through the fixation of the functional group, the fiber construct of the present invention for treating biological components develops cytokine-adsorbing ability.

The term "cytokine" refers to the cytokine likely involved in symptoms such as ulcerative colitis leukocyte, Crohn's disease, and chronic articular rheumatism to which the application of white blood cell apheresis is considered. Examples of the cytokine include interleukin-1 (IL-1), interleukin-6 (IL-6), interleukin-8 (IL-8), interleukin-10 (IL-10), tumor necrosis factor-$\alpha$ (TNF-$\alpha$), transforming growth factor beta (TGF-$\beta$), vascular endothelial growth factor (VEGF), and inhibitor apoptosis protein (IAP).

The type of the amine residue fixed in the fiber construct may be appropriately selected according to the target cytokine. When the target is interleukin-1 (IL-1), interleukin-6 (IL-6), transforming growth factor beta (TGF-$\beta$), vascular endothelial growth factor (VEGF), or inhibitor apoptosis protein (IAP), it is preferred that N,N-dimethylhexylamine, N,N-dimethyloctylamine, or N,N-dimethyllaurylamine be fixed. On the other hand, when the target is interleukin-8 (IL-8), interleukin-10 (IL-10), activated transforming growth factor beta (TGF-$\beta$), or tumor necrosis factor-$\alpha$ (TNF-$\alpha$), it is preferred that tetraethylenepentamine be fixed.

A column for treating biological components in which the fiber construct of the present invention for treating biological components is packed is preferably a cylindrical vessel. Examples of the structure include a column containing a plurality of sheets of the fiber construct; a column containing a cylindrical filter made by cylindrically rolling the fiber construct, and having an inlet and an outlet for biological components at both the ends of the filter; and a column containing a hollow cylindrical filter having sealed ends made by cylindrically rolling the fiber construct, the cylindrical vessel having a biological component outlet at a position communicating with the outer circumference surface of the hollow cylindrical filter, and a blood inlet at a position communicating with the inner circumference surface of the hollow cylindrical filter.

The fibers composing the fiber construct are preferably made of an amorphous polymer such as polystyrene or polycarbonate from the viewpoint of biocompatibility, and more preferably polystyrene for achieving good forming processability and low cost.

In order to crimp the fibers, the amorphous polymer may be mixed with a crystalline polymer (e.g., polypropylene or polyethylene terephthalate) having a melting point Tm (°C) higher than the heat treatment temperature T, followed by heat treatment. If the melting point Tm of the crystalline polymer is not higher than the heat treatment temperature T, the crystalline polymer melts and thus will not be crimped. Therefore, the above-described heat treatment must be carried out within the temperature range expressed by the following formula:

$$Tg \leq T < Tm$$

T: heat treatment temperature (°C)
Tg: glass transition temperature (°C) of amorphous polymer
Tm: melting point (°C) of crystalline polymer

The mixing ratio between the amorphous polymer and crystalline polymer for crimping the fibers means the ratio of the crystalline polymer amount to the total amount of the amorphous polymer and crystalline polymer, and is preferably from 5 to 95 wt%.

When the fiber construct in the form of nonwoven fabric is used for blood processing, too high water repellency can result in the residue of air in the nonwoven fabric to cause blood clots. Therefore, for example, when polystyrene and polypropylene are mixed, the mixing ratio between them is preferably from 5 to 20 wt%.

When heat generated during high pressure steam sterilization treatment is used for improving the production efficiency, the duration of heat treatment carried out in the temperature range expressed by the above formula is preferably 30 minutes or longer at 115 to 118°C, 15 minutes or longer at 121 to 124°C, and 10 minutes or longer at 126 to 129°C in accordance with Japanese Pharmacopoeia.

Examples of the other method for crimping the fibers include a false twisting method which includes heating and cooling the twisted threads thereby fixing the twists in the fibers, followed by twisting them in a direction opposite to the fist twisting direction; and a method of forming the treads into the shape of the gear teeth. In order to achieve an amplitude and a wavelength with a large coefficient of variation, the above-described heat treatment for the mixture of the amorphous polymer and crystalline polymer is most preferred.

EXAMPLES

The fiber construct of the present invention for treating biological components is illustrated below with reference to experimental examples.

(Making of fiber construct)

Islands-in-sea composite fibers having 36 islands, the islands being sheath-core composite fibers, were made from the following materials at a spinning rate of 800 m/minute and a stretching ratio of 3.

Island core component: polypropylene (manufactured by Grand Polymer Co., Ltd., GRAND POLYPRO J105WT, Tm: near 160°C)

Island sheath component: a melt mixture of 90 wt% of polystyrene (manufactured by PS Japan Corporation, PSJ POLYSTYRENE 685, Tg: near 100°C) and 10 wt% of polypropylene (same as above)

Sea component: copolymer polyester containing ethylene terephthalate units as the main repeating units, and 3 wt% of sodium 5-sulfoisophthalate as the copolymerization component

Mixing ratio: island core component:island sheath component:sea component=42:43:15 (weight ratio)

85 wt% of the fibers and 15 wt% of polypropylene fibers having a diameter of 20 $\mu$m were mixed, and subjected to needle-punching at a rate of 200 threads/cm$^2$, thereby making a felt (basis weight: 150 g/m$^2$). Subsequently, the felt was treated with a 3 wt% sodium hydroxide aqueous solution at 90°C to dissolve the sea component, thereby making a fiber construct (A) including a polystyrene-polypropylene mixture region as the sheath component, and having a sheath core fiber diameter of 5 $\mu$m and a bulk density of 0.02 g/cm$^3$ (total basis weight: 150 g/m$^2$). A fiber construct (C) was made in the same manner except that the needle-punching was carried out at a rate of 100 threads/cm$^2$. The fiber construct (C) included a polystyrene-polypropylene mixture region as the sheath component, and had a sheath core fiber diameter of 5 $\mu$m and a bulk density of 0.02 g/cm$^3$ (total basis weight: 150 g/m$^2$).

(Crimping)

The fiber constructs (A) and (C) were immersed in a normal saline solution, and subjected to heat treatment under four different conditions, thereby making fibers constructs (B)-1 to (B)-4 and (D) -1 to (D) -4, some fibers of which were crimped. The fiber constructs thus obtained were photographed with a scanning electron microscope (manufactured by JEOL Ltd., JSM-5400LV), and the average diameter of the fibers, the amplitude of the crimps, the wavelength of the crimps, and the coefficient of variation for the amplitude were calculated. Table 1 lists the heat treatment conditions and the amplitude of the crimps in the fiber constructs.

[Table 1]

| Fiber construct | Heat treatment temperature (°C) | Treatment time (minute) | Amplitude ($\mu$m) | Variation for the amplitude | Wavelength ($\mu$m) |
|---|---|---|---|---|---|
| (B)-1 | 117 | 105 | 53 | 0.6 | 87 |
| (B)-2 | 121 | 120 | 23 | 0.2 | 27 |
| (B)-3 | 125 | 120 | 7 | 0.1 | 20 |
| (B)-4 | 129 | 120 | 4 | 0.09 | 13 |
| (D)-1 | 115 | 55 | 190 | 0.9 | 169 |

(continued)

| Fiber construct | Heat treatment temperature (°C) | Treatment time (minute) | Amplitude (μm) | Variation for the amplitude | Wavelength (μm) |
|---|---|---|---|---|---|
| (D)-2 | 121 | 105 | 78 | 0.6 | 112 |
| (D)-3 | 128 | 105 | 22 | 0.3 | 31 |
| (D)-4 | 115 | 20 | 230 | 0.8 | 215 |

(Example 1)

From healthy volunteers, 50 mL of blood was collected into heparin (heparin concentration: 30 U/mL), and subjected to the following experiment.

The fiber constructs (B)-1 to (B)-3 were cut into disks having a diameter of 10 mm. A given number of the disks were packed into a cylindrical column having an internal volume of about 0.9 mL and a bottom diameter of 10 mm. The above-described blood, which had been mixed with lipo-polysaccharide (LPS) (LPS concentration: 70 EU/mL), was passed through the column at 37°C for 5 minutes at a flow rate of 1.33 mL/minute, and then the blood cell composition was analyzed using a multi-channel automatic blood cell analyzer. The blood cell counts thus measured were calibrated so as to make the red blood cell counts agree with each other, thereby correcting the blood cell counts of the transmitted samples. The correction values for the blood cell counts were calculated by the following formula. The experiments in Examples 1 and 2, and Comparative Examples 1 to 3 were carried out using the blood of three healthy volunteers of the same blood type.

Corrected blood cell count=blood cell count in transmitted sample x correction factor

Correction factor= (Red blood cell count in control) / (red blood cell count in transmitted sample) [0048] The removal rate of blood cells before and after passing through the columns was calculated by the following formula:

$$\text{Removal rate (\%)} = \{1 - (\text{corrected blood cell count}) / (\text{blood cell count in control})\} \times 100$$

(Example 2)

The blood cell removal rate of the fiber constructs (D)-1 to (D)-3 was determined in the same manner as in Example 1. The results are listed in Table 2.

(Comparative Example 1)

The blood cell removal rate of the fiber construct (A) was determined in the same manner as in Example 1. The results are listed in Table 2.

(Comparative Example 2)

The blood cell removal rate of the fiber construct (B)-4, which had a crimp amplitude of 5 μm or less, was determined in the same manner as in Example 1. The results are listed in Table 2.

(Comparative Example 3)

The blood cell removal rate of the fiber construct (D)-4, which had a crimp amplitude of 200 μm or more, was determined in the same manner as in Example 1. The results are listed in Table 2.
[Table 2]

| | Fiber construct | No. of sheets | Removal rate (%) | | | Remarks |
|---|---|---|---|---|---|---|
| | | | Neutrophil | Monocyte | Lymphocyte | |
| Example 1-1 | (B)-1 | 6 | 73.8 | 74.4 | 16.1 | |
| Example 1-2 | (B)-2 | 5 | 45.3 | 46.2 | 9.3 | |
| Example 1-3 | (B)-3 | 5 | 62.2 | 66.4 | 15.3 | |
| Example 2-1 | (D)-1 | 6 | 53.3 | 55.7 | 11.9 | |
| Example 2-2 | (D)-2 | 5 | 41.5 | 43.6 | 8.6 | |
| Example 2-3 | (D)-3 | 5 | 44.6 | 44.8 | 8.5 | |
| Comparative | (A) | 6 | 45.8 | 49.5 | 3 | |

| | | | | | | |
|---|---|---|---|---|---|---|
| Example 1-1 | | | | | | |
| Comparative Example 1-2 | (A) | 5 | 25.1 | 24.3 | 3 | |
| Comparative Example 2 | (B)-4 | 5 | 91.2 | 93.3 | 58.3 | Clogging occurred after a lapse of 4 minutes. |
| Comparative Example 3 | (D)-4 | 5 | 30.2 | 28.3 | 5.2 | |

According to the results in Table 2, the leukocyte removal rate of the column in which the crimped fiber construct (B) was packed (Example 1) was higher than the white blood cell removal rate of the column in which the non-crimped fiber construct (A) was packed (Comparative Example 1). This fact indicates that the fiber construct including crimped fibers has high firmness, and thus is suitable as a packing for a small column for treating blood components.

The fiber construct having a crimp amplitude outside the range of 5 to 200 $\mu$m showed marked deterioration of the leukocyte removal rate, or caused clogging (Comparative Examples 2 and 3). The fact indicates that the crimp amplitude must be 5 to 200 $\mu$m.

(Example 3)

50 mL of blood was collected into heparin (heparin concentration: 30 U/mL) from two healthy volunteers different from those in Examples 1 and 2 and Comparative Examples 1 to 3. The mixtures of the bloods were subjected to the following experiment three times.

The fiber construct (A) was cut into disks having a diameter of 10 mm, and three of the disks were packed into a cylindrical column having an internal volume of about 0.4 mL and a bottom diameter of 10 mm. 8 mL of the above-described blood, which had been mixed with LPS (LPS concentration: 70 EU/mL), was circulated at 37°C for 1 hour at a flow rate of 0.57 mL/minute; the column size used for actual treatment was downscaled with reference to the surface area, and the blood flow rate in the column was adjusted to 0.57 mL/minute, which corresponds to 50 mL/minute in the actual column (priming volume: 50 mL). Thereafter, the blood cell composition was analyzed using a multi-channel automatic blood cell analyzer. The blood cell counts thus measured were calibrated so as to make the red blood cell counts agree with each other, thereby correcting the blood cell counts of the transmitted samples. The correction values for the blood cell counts were calculated in the same manner as in Example 1. The results are listed in Table 3.

[Table 3]

|  | Removal rate (%) | | |
|---|---|---|---|
|  | Granulocyte | Monocyte | Lymphocyte |
| 1 | 39.4 | 47 | 1.54 |
| 2 | 40.3 | 47 | 3.35 |
| 3 | 67.3 | 65 | 8.84 |
| av | 49.0 | 53 | 4.58 |
| ad | 15.8 | 10 | 3.80 |

(Comparative Example 4)

ADACOLUMN (registered trademark) was disassembled, 488 beads ($3.85\ cm^3$) were taken out from the column, and packed into a cylindrical column having an internal volume of about 7.2 mL and a bottom diameter of 8 mm. The blood was circulated at 37°C for 1 hour at a flow rate of 0.42 mL/minute in the same manner as in Example 3; the column size used for actual treatment was downscaled with reference to the surface area, and the blood flow rate in the column was adjusted to 0.42 mL/minute, which corresponds to 30 mL/minute in the actual column (priming volume: 170 mL). Thereafter, the blood cell composition was analyzed. The blood was the same as that used in Example 3, and the experiment was repeated three times. The results are listed in Table 4.

[Table 4]

|  | Removal rate (%) | | |
|---|---|---|---|
|  | Granulocyte | Monocyte | Lymphocyte |
| 1 | 10.0 | 10 | 2.83 |
| 2 | 26.8 | 25 | 1.42 |
| 3 | 14.8 | 13 | 3.58 |
| av | 17.2 | 16 | 2.61 |
| ad | 8.7 | 8 | 1.10 |

The leukocyte removal rate of the mini column in which the fiber construct (A) was packed and which had a priming volume of 0.4 mL (Example 3) was higher than the leukocyte removal rate of the mini column in which the beads from ADACOLUMN (registered trademark) was packed and which had a priming volume of 7.2 mL (Comparative Example 4).

INDUSTRIAL APPLICABILITY

The present invention is applicable to a medical column for treating biological components, such as a white blood cell removal column or a cytokine adsorption column.

**Claims**

1. A fiber construct for treating biological components, comprising fibers having an average diameter of less than 50 $\mu$m, some of the fibers being crimped.

2. The fiber construct of claim 1, wherein the average diameter of the fibers is from 0.5 to 10 $\mu$m and the amplitude of the crimps is from 5 to 200 $\mu$m.

3. The fiber construct of claim 1 or 2, wherein the coefficient of variation for the amplitude is 0.1 or more.

4. The fiber construct of any one of claims 1 to 3, which has any form selected from the group consisting of nonwoven fabric, knitted fabric, woven fabric, and cotton.

5. The fiber construct of any one of claims 1 to 4, wherein the biological components are blood.

6. A column for treating biological components in which the fiber construct of any one of claims 1 to 5 is packed.

<table>
<tr><td colspan="2" align="center"><b>INTERNATIONAL SEARCH REPORT</b></td><td colspan="2">International application No.<br><b>PCT/JP2008/073685</b></td></tr>
</table>

**A. CLASSIFICATION OF SUBJECT MATTER**
*A61M1/02*(2006.01)i, *B01D15/00*(2006.01)i

According to International Patent Classification (IPC) or to both national classification and IPC

**B. FIELDS SEARCHED**

Minimum documentation searched (classification system followed by classification symbols)
A61M1/02, B01D15/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| P,X | JP 2008-652 A (Mitsubishi Paper Mills Ltd.), 10 January, 2008 (10.01.08), Par. Nos. [0022], [0024], [0039], [0093] (Family: none) | 1,2,4-6 |
| X<br>A | JP 2003-265606 A (Toray Industries, Inc.), 24 September, 2003 (24.09.03), Par. Nos. [0048], [0050] (Family: none) | 1,2,4-6<br>3 |
| A | JP 2007-70792 A (Toray Industries, Inc.), 22 March, 2007 (22.03.07), Par. No. [0005] & EP 1925701 A1 & WO 2007/018165 A1 | 1-6 |

☐ Further documents are listed in the continuation of Box C.   ☐ See patent family annex.

| | |
|---|---|
| \* Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>20 January, 2009 (20.01.09) | Date of mailing of the international search report<br>03 February, 2009 (03.02.09) |
|---|---|
| Name and mailing address of the ISA/<br>Japanese Patent Office | Authorized officer |
| Facsimile No. | Telephone No. |

Form PCT/ISA/210 (second sheet) (April 2007)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 60193468 A
- JP 5168706 A
- JP 10225515 A
- JP 12237585 B
- JP 2002113097 A